(19) Europäisches Patentamt / European Patent Office / Office européen des brevets

(11) **EP 1 728 507 B2**

(12) **NEW EUROPEAN PATENT SPECIFICATION**
After opposition procedure

(45) Date of publication and mention of the opposition decision:
**12.11.2014 Bulletin 2014/46**

(45) Mention of the grant of the patent:
**16.03.2011 Bulletin 2011/11**

(21) Application number: **06076172.3**

(22) Date of filing: **06.06.2006**

(51) Int Cl.:
***A61K 31/122*** (2006.01) ***A61K 31/593*** (2006.01)
***A23L 1/302*** (2006.01) ***A23L 1/303*** (2006.01)
***A61P 9/10*** (2006.01)

(54) **USE OF VITAMIN K FOR REVERSING CALCIFICATION OF BLOOD VESSELS**

ZUSAMMENSETZUNGEN ZUR BEHANDLUNG UND VORBEUGUNG VON KARDIOVASKULÄREN ERKRANKUNGEN

COMPOSITIONS POUR LE TRAITEMENT OU LA PREVENTION DES MALADIES CARDIOVASCULAIRES

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.06.2005 US 144853**

(43) Date of publication of application:
**06.12.2006 Bulletin 2006/49**

(73) Proprietor: **NattoPharma ASA**
**0230 Oslo (NO)**

(72) Inventor: **Vermeer, Cees**
**6200 MD Maastricht (NL)**

(74) Representative: **Weiss, Wolfgang**
**Weickmann & Weickmann**
**Patentanwälte**
**Richard-Strauss-Strasse 80**
**81679 München (DE)**

(56) References cited:
**EP-A- 0 679 394** **EP-A2- 0 679 394**
**WO-A-2004/019923** **WO-A1-2004//019923**
**WO-A2-03//013420**

- **SEYAMA YOSHIYUKI ET AL: "Effect of vitamin K-2 on experimental calcinosis induced by vitamin D-2 in rat soft tissue" INTERNATIONAL JOURNAL FOR VITAMIN AND NUTRITION RESEARCH, vol. 66, no. 1, 1996, pages 36-38, XP009072199 ISSN: 0300-9831**
- **BERKNER K L ET AL: "The physiology of vitamin K nutriture and vitamin K-dependent protein function in atherosclerosis." JOURNAL OF THROMBOSIS AND HAEMOSTASIS : JTH. DEC 2004, vol. 2, no. 12, December 2004 (2004-12), pages 2118-2132, XP009072206 ISSN: 1538-7933**
- **GELEIJNSE JOHANNA M ET AL: "Dietary intake of menaquinone is associated with a reduced risk of coronary heart disease: The Rotterdam Study" JOURNAL OF NUTRITION, vol. 134, no. 11, November 2004 (2004-11), pages 3100-3105, XP002399143 ISSN: 0022-3166 & BERKNER K L ET AL: "The physiology of vitamin K nutriture and vitamin K-dependent protein function in atherosclerosis." JOURNAL OF THROMBOSIS AND HAEMOSTASIS : JTH. DEC 2004, vol. 2, no. 12, December 2004 (2004-12), pages 2118-2132, ISSN: 1538-7933**
- **KAWASHIMA H ET AL: "Effects of vitamin K2 (Menatetrenone) on atherosclerosis and blood coagulation in hypercholesterolemic rabbits" JAPANESE JOURNAL OF PHARMACOLOGY, THE JAPANESE PHARMACOLOGICAL SOCIETY, KYOTO, JP, vol. 75, no. 2, October 1997 (1997-10), pages 135-143, XP009020469 ISSN: 0021-5198**
- **S. YOSHIYUKI ET AL.: 'Effect of Vitamin K2 on Experimental Calcinosis Induced by Vitamin D2 in Rat Soft Tissue' INTERNATIONAL JOURNAL FOR VITAMIN AND NUTRITION RESEARCH vol. 66, 1996, pages 36 - 38**
- **K.L. BERKNER ET AL.: 'The physiology of vitamin K nutriture and vitamin K-dependent protein function in atherosclerosis' JOURNAL OF THROMBOSIS AND HAEMOSTASIS vol. 2, 2004, pages 2118 - 2132**



Printed by Jouve, 75001 PARIS (FR)

**(Cont. next page)**

- **J.M. GELEIINSE ET AL.: 'Dietary Intake of Vitamin K-2 may Protect Agains Ischaemic heart Disease: The Rotterdam Study' SUPPLEMENT TO THE JOURNAL THROMBOSIS AND HAEMOSTASIS July 2001, pages 2 - 26**
- **'Medical Dictionary Extracts', W.B. SAUNDERS COMPANY page 1736**
- **H.M.H. SPRONK ET AL.: 'Tissue-Specific Utilization of Menaquinone-4 Results in the Prevention of Arterial Calcification in Warfarin-Treated Rats' JOURNAL OF VASCULAR RESEARCH vol. 40, 19 March 2003, pages 531 - 537**
- **K-S G. JIE ET AL.: 'Vitamin K intake and osteocalcin levels in women with and without aortic atherosclerosis: a population-based study' ATHEROSCLEROSIS vol. 116, 01 February 1995, pages 117 - 123**
- **Declaration by Prof. Vermeer of 17.09.2008**
- **Harlan Europe online catalogue, "Sprague Dawley"**
- **Tatonic online catalogue, "Sprague Dawley® Rat Model"**


Remarks:

The file contains technical information submitted after the application was filed and not included in this specification

## Description

Field of the Invention

The present invention concerns the use of vitamin K to reverse calcification of blood vessels.

Background of the Invention

The process of aging is associated with irreversible physiological changes to the circulatory system, leading to an increased risk of blood pressure disorders, Coronary Heart Disease (CHD), and stroke. For women, this risk rises dramatically after the onset of menopause. These conditions have a significant impact on quality of life for the middle-aged and elderly and account for a large proportion of deaths and chronic illnesses in modern societies.

Patients suffering from cardiovascular disorders are frequently prescribed anticoagulants, antihypertensives, cholesterol-lowering medications, and the like. These medications usually present harmful side-effects or health risks, and moreover, the chronic effects of taking such medication regularly over the course of years or decades are not well studied. As life expectancies increase, it would be desirable to find long-term, safe and reliable natural therapies to prevent, treat or even reverse the consequences of aging on the vasculature.

It has long been recognized that vitamin K is an essential component of the diet. It was first identified as an element needed to prevent haemorrhaging by activating blood-clotting factors. Natural K-vitamers are menadione-derivatives differing from each other in the polyisoprenoid side chain attached to the 3-position of the ring structure. Vitamin K can be provided in the diet by dark green, leafy vegetables ($K_1$ or phylloquinone), and by fermented foods such as cheese and curd ($K_2$ or menaquinone). $K_2$ vitamins are also synthesized in the small intestine by resident symbiotic bacteria. Vitamin K is also needed for carboxylation of two bone matrix proteins necessary for normal bone metabolism.

In EP-A-0 679 394 and likewise in Jpn. J. Pharmacol. (1997) 75:135-143 it is disclosed that a high dietary intake of vitamin K and related molecules can reduce further arterial calcification, but not reverse it, from which it is concluded that arteriosclerosis can be treated using vitamin K. Arteriosclerosis is a disease of the arteries characterized by inflammation, macrophage invasion, foam cell formation, intima thickening, accretion of cholesterol, and formation of an atherosclerotic plaque, which over time can become calcified. The onset of atherosclerosis is invariably in the large arteries such as for example, the aorta and coronary arteries. In more advanced stages one may see plaque rupture leading to sudden vascular occlusion, myocardial infarction and cerebrovascular accident (infarction of the brain).

A completely different process is that of vascular stiffening due to loss of elasticity of the arteries. Vascular stiffening is associated with ageing, diabetes mellitus and renal dysfunction; it is the result of degradation of the elastic lamellae in the tunica media resulting in loss of elasticity. The onset of vascular stiffening is generally seen in the smaller vessels, but extends to the larger arteries. This will lead to increased blood pressure, vascular widening, and in later stages to rupture of (mainly the small) arteries and capillaries.

WO 2004/019923 discloses that vitamin K is effective in counter acting the reduction in arterial elasticity normally associated with aging.

Studies have shown that also on a molecular level age-related stiffening of the arteries can be distinguished from arteriosclerotic/atherosclerotic calcification.

There is a need for effective compositions and methods for reversing calcification of blood vessels.

Summary of the Invention

In one aspect, the present invention provides the use of vitamin K for the preparation of a pharmaceutical or nutritional formulation for reversing calcification of a blood vessel in a mammal suffering from calcification of a blood vessel, when administration to the mammal an effective amount of said vitamin K to reverse calcification of a blood vessel.

It has specifically been discovered that high intake of vitamin K can lead to removal of calcified precipitates from blood vessels that have already been affected by pre-existing calcification. This is a new and stunning discovery with great importance for patients with existing artery disease. Implications for the nutrition industry are that vitamin K-enriched foods and food supplements may be developed.

Calcification of a blood vessel is associated with a disease selected from the group consisting of arteriosclerosis, including Mönckeberg's sclerosis, osteoarthritis, inflammation-induced calcification, including Bechterev's disease, tumor-induced calcification, skin calcification, including pseudo-xanthoma elasticum (PXE), and calcifylaxis in end-stage renal disease.

In a further aspect, the invention provides a composition for promoting healthy arteries, comprising vitamin K or a derivative thereof, and optionally vitamin D or a derivative thereof, and one or more additional components selected from: polyphenols, vitamin C, vitamin E (tocopherols and/or tocotrienols), L-Arginine, phytosterols, antihypertensive peptides, soluble fibers (e.g. guar, pectin), omega-3, omega-6 and/or omega-9 fatty acids, carnitine, taurine, coenzyme

Q10, creatine, folic acid, folates, magnesium, potassium, vitamin B6, and vitamin B12.

In yet another aspect of the invention there is provided a kit comprising Vitamin K or a derivative thereof, and optionally vitamin D or a derivative thereof and a medicament, for simultaneous, separate or sequential administration, wherein said medicament is selected from the group consisting of: anticoagulants, antithrombotics, fibrinolytics, antihypertensives, diuretics, antianginals, hypolipidaemic agents, beta-blockers, ACE inhibitors, cardiac glycosides, phosphodiesterase inhibitors, antiarrhythmics, and calcium antagonists.

In another aspect of the invention, a composition is provided for reversing calcification of a blood vessel in a human, comprising about 50 micrograms to 50 milligrams of vitamin K .

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description and appended claims.

## Brief Description of the Figures

Figure 1 shows how the Distensibility Coefficient (DC) varies over a 3 year study period when placebo, Vitamin D (MD) and Vitamins K plus D (MDK) are administered to a group of postmenopausal women.

Figure 2 shows how the Compliance Coefficient (CC) varies over a 3 year study period when placebo, Vitamin D (MD) and Vitamins K plus D (MDK) are administered to a group of postmenopausal women.

In each case the black bar represents the baseline measurement (100%), and the shaded bars are the % change relative to baseline after 3 years.

Figure 3 is a graph showing the data of 18 rats on a control diet (containing 5 μg/g of vitamin K). Six rats were killed at baseline, six after 6 weeks, and six after 12 weeks. Apart from the cellular calcium, no accretion of calcium was observed during the test period.

Figure 4 is a graph showing results obtain when rats received a 6-week treatment with the vitamin K-antagonist warfarin (indicated as "low K") in a protocol that does not influence blood clotting, but mainly affects bone and vascular vitamin K status. Six rats were killed after 6 weeks of treatment, and another six after 12 weeks of treatment. As shown, there was a constant increase of calcium in the arterial wall.

Figure 5 is a graph showing results obtain after rats were treated with warfarin for six weeks and subsequently fed a standard diet containing 5 μg vitamin K per g of food (indicated as "norm K"). The calcification did not stop upon using the normal diet.

Figure 6 is a graph showing results obtain after rats were treated with warfarin for six weeks and subsequently received for another six weeks, a high vitamin K diet. One group received a diet containing 100 μ/g of vitamin K1, the other group received a diet containing 100 μg/g of vitamin K2 (menaquinone-4). These dosages represent about 20-fold the normal vitamin K intake for rats. The most surprising finding was that the pre-formed calcium crystals had dissolved to a great deal as indicated by the decline in aortic calcification.

## Detailed Description of the Invention

For the purposes of this specification and appended claims, unless otherwise indicated, all numbers expressing quantities of ingredients, percentages or proportions of materials, reaction conditions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters set forth, the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, e.g., 5.5 to 10.

It is noted that, as used in this specification and the appended claims, the singular forms "a," "an," and "the,"

include plural referents unless expressly and unequivocally limited to one referent. Thus, for example, reference to "a monomer" includes two or more monomers.

Reference will now be made in detail to certain embodiments of the invention, examples of which are illustrated in the accompanying drawings. While the invention will be described in conjunction with the illustrated embodiments, it will be understood that they are not intended to limit the invention to those embodiments. On the contrary, the invention is intended to cover all alternatives, modifications, and equivalents, which may be included within the invention as defined by the appended claims.

Preferably vitamin K and derivatives refers to one or more compounds of Formula 1', and/or their pharmaceutically or nutritionally acceptable salts,

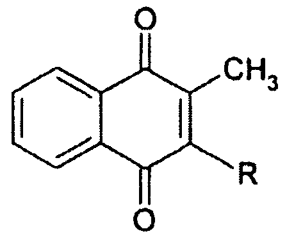

Formula 1'

where R may be any covalently linked organic group including polyisoprenoid residues, esters, ethers, thiol adducts, etc. and especially the compounds thereof of Formula 2:

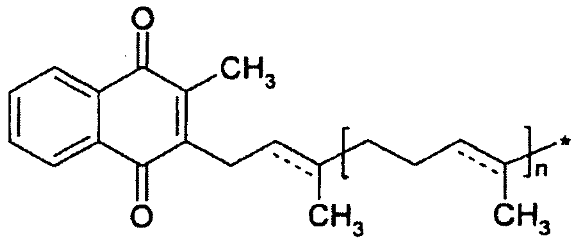

Formula 2

in which n is an integer from 1 to 12; and in which the broken lines indicate the optional presence of a double bond.

Vitamin K and derivatives thereof, as used herein, refers in particular to phylloquinone (also known as vitamin $K_1$), dihydrophylloquinone; menaquinone-4 (MK-4) and the long chain menaquinones. It is generally accepted that the naphthoquinone is the functional group, so that the mechanism of action is similar for all K vitamins. Differences may be expected, however, with respect to intestinal absorption, transport, tissue distribution, and bioavailability. For use in the present invention, phylloquinone and MK-4 are preferred, and phylloquinone is particularly preferred.

Sources of vitamin K which can be used according to the present invention include the following: phylloquinone from natural sources such as vegetable extracts, fats and oils, synthetic phylloquinone, synthetic vitamin $K_3$ (menadione), different forms of vitamin $K_2$: synthetic MK-4, MK-5, MK-6, MK-7, MK-8, MK-9, MK-10, MK-11, MK-12 and MK-13, natto (food prepared from fermented soy-bean, rich in MK-7), and other fermented foods or dairy products.

Vitamin K enriched nutritional products can be manufactured to provide the daily requirements of vitamin K. For example, vitamin K can be added to food products, such as for example, meal replacers, ice cream, sauces, dressings, spreads, bars, sweets, snacks, cereals, beverages, etc. by methods as described in EP 1,153,548. Also, vitamin K can be used in food supplements such as multivitamins, tablets, capsules, and other forms.

The dose of vitamin K useful in performing the invention is not restricted but varies depending on, for example, the age of the subject and the degree of risk of developing arterial stiffening or the degree of calcification of the blood vessel. Current AI values or Adequate Intakes (as determined by the Institute of Medicine) are 120 μg for men and 90 μg for women. Benefits may be derived by selecting dosages higher than the AI values, particularly in population groups where vitamin K deficiencies are common, for instance among postmenopausal women. For example, suitable dosages may lie in the range 10 to 1000 μg, more preferably 50 to 500 μg, and most preferably 100 to 200 μg vitamin K/day. Where national legislation permits, it may be advisable to provide dosage ranges as high as from 1 to 200 mg/day, preferably from 5 to 150 mg/day, and more preferably from 10 to 100 mg/day.

In various embodiments of the present invention, high vitamin K intake induced the removal of pre-existing

calcium deposits within the blood vessel wall. These doses in humans would be between 50 μg/day to 2 mg/day.

In terms of body weight, daily dosage may vary between 0.5 to 200 μg/kg body weight/day, preferably 0.75 to 25 μg/kg body weight/day, more preferred 1 to 15 μg/kg body weight/day.

As used herein, the term "effective amount" or "therapeutically effective amount" are interchangeable and refer to an amount that results in an improvement, reversal or remediation of the symptoms of the disease or condition. For example, an effective amount of vitamin K to remove calcium, reduce or reverse calcification of a blood vessel in humans can be between about 50 micrograms/day and an upper limit of about 50 milligrams/day. In various embodiments, to remove calcium, reduce or reverse calcification of a blood vessel in humans, a dose between about 100 micrograms and about 2 milligrams/per day is preferred. These doses are particularly useful in treating or reversing diseases such as, for example, arteriosclerosis.

Vitamin D is included together with vitamin K in the composition used in the clinical study, and may play a role in supporting the function of vitamin K in preventing arterial stiffening. Any form of natural or synthetic vitamin D may be employed, including vitamin $D_1$, vitamin $D_2$ (calciferol), vitamin $D_3$ (cholecalciferol) and vitamin D analogues (e.g. alfacalcidol, dihydrotachysterol, calcitriol). Natural sources of vitamin D include saltwater fish, organ meats, fish-liver oils and egg yolk. Suitable dosages of vitamin D are 2 to 50 μg/day, preferably 5 to 20 μg/day, and most preferably about 7 to 10 μg/day.

In the clinical study described in the Example 1, arterial wall property measurements were taken at t=0 and t=3 years. This is good support for concluding that ingestion of vitamin K over long periods is an effective way of limiting an increase in arterial stiffness. The preferred treatment period is a minimum of 6 months, more preferably at least 12 or 18 months, and ideally at least 36 months. In fact, as there are no adverse side-effects associated with dietary vitamin K supplementation, it should be regarded as an essential component of a healthy lifestyle over the course of a lifetime, and especially throughout middle age and old age.

The preferred route of administration of vitamin K is enterally, especially orally, but the parenteral or topical routes are viable alternatives. "Oral administration" as used herein includes oral, buccal, enteral or intragastric administration. The term "parenteral administration" as used herein includes any form of administration in which the vitamin K is absorbed into the blood stream without involving absorption via the intestines. Exemplary parenteral administrations that are used in the present invention include, but are not limited to intramuscular, intravenous, intraperitoneal, intraocular, subcutaneous or intraarticular administration.

Vitamin K is conventionally provided in the form of tablets or capsules, i.e. in a pharmaceutical or dietary supplement format. For pharmaceutical preparations or dietary supplements the vitamin K may be compounded with pharmaceutically acceptable carriers, excipients or diluents in the forms of pills, tablets (coated or uncoated), hard or soft capsules, dragées, lozenges, oral solutions, suspensions and dispersions, syrups or sterile parenteral preparations. Suitable excipients include inert diluents such as calcium carbonate, sodium carbonate, lactose, calcium phosphate, sodium phosphate; granulating and disintegrating agents such as cornstarch or alginic acid; binding agents such as starch gelatin or acacia; effervescents; and lubricating agents such as magnesium stearate, stearic acid or talc.

It is also possible to deliver or administer Vitamin K (optionally together with vitamin D) in a fortified food or beverage product. Preferred nutritional product formats include: juice drinks, dairy drinks, powdered drinks, sports drinks, mineral water, soy beverages, hot chocolate, malt drinks, biscuits, bread, crackers, confectioneries, chocolate, chewing-gum, margarines, spreads, yoghurts, breakfast cereals, snack bars, meal replacements, protein powders, desserts, and medical nutrition tube feeds and nutritional supplements.

Conventional additives may be included in the compositions of the invention, including any of those selected from preservatives, chelating agents, effervescing agents, natural or artificial sweeteners, flavoring agents, coloring agents, taste masking agents, acidulants, emulsifiers, thickening agents, suspending agents, dispersing or wetting agents, antioxidants, and the like.

"Elevated blood pressure" or "hypertension" as used herein refers to a blood pressure persistently exceeding 140/90 mmHg (systolic/diastolic).

In various embodiments of the present invention, vitamin K induces the removal of pre-existing calcium deposits from the mammal. Blood vessels are made of three layers, called from the luminal side outward, the tunica intima, the tunica media and the tunica adventitia. Calcification can occur in or on any of these layers. Typically, calcification of the blood vessel makes the vessel wall rigid, fragile and subject to rupture. For purposes of the present invention, blood vessels include capillaries, veins, arteries, venules, and/or arterioles.

Calcification includes the abnormal deposition of calcium crystals at sites within and/or on the blood vessels. Pre-existing calcification can occur in diseases such as, for example, arteriosclerosis where atherosclerotic plaque that has been calcified can partially or completely occlude the blood vessels. In various stages of atherosclerosis not only the plaque, but also the vascular tissue surrounding the plaque may calcify.

For the present application, arteriosclerosis also includes Mönckeberg's sclerosis. Other diseases associated with pathological calcification, include but are not limited to, calcification of cartilage (osteoarthritis), inflammation-induced calcification (e.g. in Bechterev's disease), tumor-induced calcification (often seen in breast cancer), skin calcification

such as in pseudoxanthoma elasticum (PXE), and calcifylaxis in end-stage renal disease.

Often non-drug/nutritional treatment modalities for arteriosclerosis involve catheter-based procedures, such as angioplasty, that use a catheter inserted into an artery to press the plaque against the walls of the arteries to increase space for blood to flow. Stenting, usually done after angioplasty, uses a wire mesh tube placed in the damaged artery to support the arterial walls and is used to keep the vessel open. Atherectomy can be performed where instruments inserted via a catheter are used to cut away and remove plaque so that blood can flow more easily.

Surgical procedures are also performed to treat atherosclerosis. These include endarterectomy or the removal of the lining of an artery obstructed with large plaques. Arterioplasty, another type of surgery, involves the repair of an aneurysm using synthetic tissue. Bypass surgery may also be performed where blood flow is restored using blood vessels obtained from different areas of the body.

In various embodiments of the present invention, by administering vitamin K, the need for catheter-based or surgical treatment of atherosclerosis can be reduced or avoided. Alternatively, the vitamin K can be administered as part of a comprehensive arteriosclerosis treatment plan with the surgical and/or catheter-based procedures to remove calcification.

Preferably, there is removal of from about 10% to about 100% of abnormal calcium deposits from the blood vessel. The preferred treatment period for removal of calcification can be at a minimum of 6 -12 weeks, preferably at least 6 to 8 months and most preferably at least 12 months or longer. In various embodiments, the treatment period can be life long for the subject.

Clinically, calcification can be detected by, for example, thallium stress testing, radiography, coronary calcification scans, fluoroscopy, CT, angioplasty, MRI imaging, sonography, biopsy, by histochemistry or the like.

The term "reducing calcification" or reduction of calcification includes decreasing the rate of calcification in and/or on the blood vessel. As used herein, the term "reversing calcification" includes removing pre-existing calcium deposited in and/or on the blood vessel. While the mechanism is not completely understood, it is believed that vitamin K enhances MGP (matrix Gla-protein) to rigorously protect against further accretion of calcium, whereas other proteins or cells (macrophages, osteoclast-like smooth muscle cells, or others) participate in the actual removal of the calcium. Alternatively, MGP's Gla-residues (formed under vitamin K influence) have some structural resemblance to EDTA, and may act as such to directly dissolve the calcium. Finally, it may be that MGP (through its Gla-residues) may directly bind to the calcium precipitates (as all Gla-proteins do) and exert chemo-attractant activity towards macrophages, which are then thought to remove the calcium-MGP complexes.

In various embodiments of the present invention, it has been discovered that vitamin K can be used to treat and/or reverse arteriosclerosis in a mammal. Typically, arteriosclerosis is a disease of the arteries characterized by inflammation, macrophage invasion, foam cell formation, intima thickening, accretion of cholesterol, and formation of an atherosclerotic plaque, which over time can become calcified. The onset of atherosclerosis is invariably in the large arteries such aorta and coronary arteries. In more advanced stages one may see plaque rupture leading to sudden vascular occlusion, myocardial infarction and cerebrovascular accident (infarction of the brain).

In various embodiments, the vitamin K is administered to a mammal suffering from a pre-existing cardiovascular disease that requires treatment. Mammals include, for example, humans, as well as pet animals such as dogs and cats, laboratory animals, such as rats and mice, and farm animals, such as sheep, horses and cows.

Having now generally described the invention, the same may be more readily understood through the following reference to the following examples.

## EXAMPLES

<u>Example 1</u>

<u>Clinical Study to compare effects of supplementation of Vitamin D and Vitamin D + Vitamin K on a group of healthy postmenopausal women.</u>

<u>Subjects</u>

The participants were enrolled in a 3-year double-blind placebo-controlled clinical trial in which the effects of minerals, vitamin D- and vitamin K-containing supplements were investigated on bone mineral density and vessel wall characteristics. Inclusion criteria were: apparently healthy women, Caucasian, between 50 and 60 years old, and at least 2 years postmenopausal. Exclusion criteria were: use of oral anticoagulants, corticosteroids, hormone replacement therapy, vitamin concentrates or food supplements, and high alcohol consumption (> 6 glasses/day). In total 181 women met the criteria for participation and were randomized into the study. Information on cardiovascular risk factors, current health status, medical history, drug use and smoking behaviour was collected before the start of the study. Within this trial participants underwent clinical examinations at 0, 3, 12, 18, 24 and 36 months. The vascular examinations took

place at baseline and at the end of the study after 3 years.

All participants gave written informed consent and the trial was approved by the Maastricht University Hospital Medical Ethics Committee.

Study Design

The subjects were randomized into three groups. In the first group (n=60) participants received a placebo (maltodextrin), in the second group (n=58) participants received a supplement containing 500 mg calcium (natural calcium complex derived from milk), 10mg zinc, 150 mg magnesium and 8 $\mu$g vitamin $D_3$ (minerals + vitamin D = MD-group), and in the third group (n=63) participants received a supplement containing the same constituents as the MD group but with an additional 1 mg of vitamin $K_1$ (minerals + vitamins D+K = MDK-group). The three different types of supplements were similar in appearance and taste, and participants were allowed to choose between a supplement in the form of a tasteless powder (to be mixed with water before intake) or in the form of chocolate-coated tablets with a crunchy malt core. Participants were instructed to take one sachet with powder or three tablets per day during evening hours, preferably after the meal. Also, they were advised to maintain their usual diets and to avoid taking supplements containing either calcium, vitamin D, or vitamin K for two months before and throughout the study. Novartis Consumer Health SA (Nyon, Switzerland) prepared and provided all supplements.

The right common carotid artery of each patient was investigated. The same investigator performed all examinations at the start and the end of the study and for each participant several repeated measurements (5-7) are made during one session. Reproducibility was evaluated for assessment of common carotid artery distension and diameter.

Before the vascular examination, height and weight were measured with standardized equipment to estimate the body mass index (weight/height$^2$).

Measurements

The primary outcome measures for the purposes of this study were the vessel wall characteristics of the common carotid artery measured with ultrasound (ATL Mark V).

The ultrasonic vessel wall tracking system (WTS) to determine arterial wall properties has been described in detail before (Hoeks AP et al., Ultrasound Med Biol 1990; 16:121-8, and Kool MJF et al., Cardiovascular Research 1994;28:610-614). This ultrasound system provides estimates of the arterial end-diastolic diameter (d) and the change in diameter from diastole to systole ($\Delta$d) normalized for the end-diastolic diameter ($\Delta$d/d) for each captured heart beat. In parallel with diameter change measurement, arterial blood pressure was recorded at the level of the brachial artery by means of a semiautomated oscillometric device (DINAMAP). Pulse pressure ($\Delta$p), defined as systolic minus diastolic blood pressure, was determined by averaging the three measurements nearest to the distension measurements. From d, $\Delta$d and $\Delta$p, vascular distensibility (DC) and compliance (CC) were calculated according to the following equations:

$$DC = (2d\Delta d + \Delta d^2)/(d^2/\Delta p) \quad \text{(Distensibility Coefficient)}$$

$$CC = \pi(2d\Delta d + \Delta d^2)/4\Delta p \quad \text{(Compliance Coefficient)}$$

The intima-media thickness (IMT) of the posterior wall was measured simultaneously at the same location (2-3 cm proximal to the bifurcation) of the common carotid artery where the diameter and diameter changes were measured. At the end of the session, recorded IMT-files are processed employing the wall thickness program. The threshold for the derivative was maintained at 0.025. Each heart-beat within a recording resulted in an estimate of wall thickness; the median of the estimates per recording was used for further evaluation.

Statistical Analysis

Statistical analysis was performed using the Statistical Package SPSS (SPSS Corp, Chicago, IL). Results are presented as means ± standard deviation (SD), unless indicated otherwise. Only participants who had completed the study were included in the analysis. Furthermore, participants who during the study had started to use medications which are known to have a direct effect on the vessel wall, were excluded from analysis. Also, participants with atherosclerotic plaques in the common carotid artery and a high variability in the results (arterial translation of > 2 mm and beat-to-beat variation in distension of > 20%) were excluded.

A paired t-test was used to evaluate the change in the vessel wall characteristics over the three years within each group. We considered a level of $p<0.05$ to be statistically significant. For every participant, the percentage change from baseline in all parameters was calculated and the mean change from baseline was calculated per group. Primary outcome analysis consisted of comparison of the change in DC, CC, PP and IMT between the MD-group and placebo and between the MDK-group and placebo. Linear regression analysis was used with the change in vascular parameters relative to baseline as dependent variable and the treatment groups and several covariates as explanatory variables. Baseline values of age, BMI, smoking (yes or no), heart rate and mean arterial pressure were chosen as covariates, because their influence on the change in vascular properties or response to the supplementation could not be excluded.

Vascular parameters of elasticity

Table 1 details the baseline measurements of each study group. Table 2 summarizes per group the differences between the mean values at baseline and at the end of the study for all vascular parameters with their paired-levels of significance. As was to be expected, the DC and CC in the placebo group decreased significantly (by 10% and 6%, respectively). The PP, on the other hand, increased by 7%, but the increase did not reach the level of significance. In the MD-group, DC decreased significantly (by 7%) and CC decreased by 4%, while the PP increased by 6%; however these latter two changes did not reach the level of significance. In the MDK-group, however, the DC and CC remained approximately constant over the three year period, the CC even showing a tendency to increase (+3%). The PP remained unchanged throughout the entire study period.

Figures 1 and 2 (see also Table 3 and Table 3a) illustrate the percentage change in DC and CC respectively of the three groups. After adjustment for baseline heart rate, mean arterial pressure, age, weight and smoking, the changes in the placebo-relative to the MDK-group remained statistically significant and were: 8.8% decrease of DC (95% Cl: 1.9 to 21.4), 8.6% decrease of CC (95% Cl: 1.8 to 20.3), and 6.3% increase of PP (95% Cl: -17.1 to -0.7). In the same analysis no differences were found between the changes in the placebo- and the MD-group: 2.5% decrease of DC (95% Cl: -14.8 to 6.3), 2.2% decrease of CC (95% Cl: -13.8 to 6.3), and 0.11 % increase of PP (95% Cl: -5.6 to 12.1).

Discussion of Results

The deleterious effects on the arteries of aging over a period of 3 years are clearly evident from the control (placebo) group, and underline how rapidly the vasculature can go into decline. The medical practitioner, being aware of the link between a decrease in elasticity of the arteries and diverse cardiovascular conditions, would recognise from these data that there is an urgent need to find a treatment method capable of combating the rapid decline in arterial elasticity, particularly in postmenopausal women.

The MD group, who received a vitamin D supplement, failed to show any improvement in measures of vascular wall aging relative to the placebo group. It can be concluded that provision of vitamin D alone is not capable of delivering cardiovascular benefits to postmenopausal women fulfilling the criteria applied in the present study.

In stark contrast to the placebo and MD groups, the MDK group showed significant relative improvements in distensibility, compliance and pulse pressure over the 3 year period of the study. These results demonstrate that regular consumption of vitamin K, or of the combination of vitamin K and vitamin D, can slow and maybe even reverse the process of stiffening of the arteries. As a consequence of slowing down the process of arterial stiffening, vitamin K supplementation inevitably impacts on the incidence of cardiovascular disorders linked to arterial stiffening, including those related to increased strain on the heart and reduced responsiveness of the circulatory system to changes in demand.

Table 1

| Baseline characteristics (mean ± standard deviation) in the three treatment groups | | | |
|---|---|---|---|
| **Baseline-characteristics** | **Placebo** (n=40)<br>Mean ± SD | **MD-group** (n=30)<br>Mean ± SD | **MDK-group** (n=38)<br>Mean ± SD |
| Age (yr) | 54.1 ± 3.0 | 55.9 ± 2.8* | 55.4 ± 2.8 |
| Weight (kg) | 69.5 ± 11.9 | 70.6 ± 11.1 | 66.3 ± 9.5 |
| Height (m) | 1.65 ± 0.05 | 1.65 ± 0.07 | 1.63 ± 0.06 |
| BMI ($kg/m^2$) | 25.6 ± 4.3 | 26.0 ± 4.4 | 25.1 ± 3.1 |
| Postmenopausal age (yr) | 4.6 ± 3.7 | 7.6 ± 5.1** | 5.1 ± 4.3 |
| Non-smokers (%) | 75.0 | 73.9 | 85.0 |

(continued)

| Baseline characteristics (mean ± standard deviation) in the three treatment groups | | | |
|---|---|---|---|
| **Baseline-characteristics** | **Placebo** (n=40) Mean ± SD | **MD-group** (n=30) Mean ± SD | **MDK-group** (n=38) Mean ± SD |
| Diameter ($\mu$m) | 7162 ± 562 | 7314 ± 582 | 7173 ± 411 |
| Distension ($\mu$m) | 372 ± 118 | 353 ± 83 | 332 ± 83 |
| Pulse Pressure (mmHg) | 51.9 ± 11.1 | 52.9 ± 10.1 | 53.7 ± 14.3 |
| Heart Rate (beats/min) | 60.8 ± 9.2 | 63.1 ± 8.9 | 60.6 ± 6.6 |
| CC ($mm^2$/kPa) | 0.64 ± 0.23 | 0.61 ± 0.20 | 0.56 ± 0.17 |
| DC ($MPa^{-1}$) | 15.8 ± 5.2 | 14.5 ± 4.0 | 14.0 ± 4.0 |
| IMT (mm) | 0.63 ± 0.11 | 0.64 ± 0.10 | 0.61 ± 0.08 |
| * significant different from placebo (p<0.05)<br>** significant different from placebo and MDK-group (p<0.05) | | | |

Table 2

| Change in vessel wall characteristics (mean ± SD) in study population after 3 years | | | |
|---|---|---|---|
| Vessel wall characteristics | **Placebo** (n=40)<br>Difference between T=0 and T=3 years (paired t-test) | **MD-group** (n=30)<br>Difference between T=0 and T=3 years (paired t-test) | **MDK-group** (n=38)<br>Difference between T=0 and T=3 years (paired t-test) |
| Diameter ($\mu$m) | 196 ± 295 (p=0.00) | 154 ± 179 (p=0.00) | 131 ± 226 (p=0.00) |
| Distension ($\mu$m) | -21 ± 61 (p=0.03) | -12.6 ± 47 (p=0.15) | -3.9 ± 49 (p=0.63) |
| Pulse Pressure (mm Hg) | 2.7 ± 9.9 (p=0.09) | 2.8 ± 10.1 (p=0.14) | -0.18 ± 7.6 (p=0.89) |
| DC ($MPa^{-1}$) | -1.8 ± 3.4 (p=0.00) | -1.4 ± 3.0 (p=0.02) | -0.39 ± 3.0 (p=0.43) |
| CC ($mm^2$/kPa) | -0.05 ± 0.1 (p=0.01) | -0.04 ± 0.11 (p=0.10) | 0.01 ± 0.11 (p=0.75) |
| IMT (mm) | 0.05 ± 0.08 (p=0.00) | 0.02 ± 0.09 (p=0.32) | 0.06 ± 0.06 (p=0.00) |
| Heart rate (beats/min) | 3.0 ± 7.0 (p=0.01) | | |

Table 3

| Mean % change from baseline in vessel wall characteristics.<br>(for each subject the % change from baseline is calculated for each variable and then the mean of these individual changes is calculated per group) | | | |
|---|---|---|---|
| Vessel wall characteristics | **Placebo** (n=40) Mean % change from baseline | **MD-group** (n=30) Mean % change from baseline | **MDK-group** (n=38) Mean % change from baseline |
| Diameter ($\mu$m) | 2.8% ± 4.1 | 2.2% ± 2.5 | 1.8% ± 3.1 |
| Distension ($\mu$m) | -4.3% ± 15.9 | -2.4% ± 13.0 | 0.3% ± 15.9 |
| Pulse Pressure (mm Hg) | 6.5% ± 19.7 | 6.3% ± 20.0 | 0.2% ± 13.4* |
| DC ($MPa^{-1}$) | -9.6% ± 21.4 | -7.1 % ± 18.3 | -0.8% ± 21.9* |

(continued)

| Mean % change from baseline in vessel wall characteristics. (for each subject the % change from baseline is calculated for each variable and then the mean of these individual changes is calculated per group) | | | |
|---|---|---|---|
| Vessel wall characteristics | **Placebo** (n=40) Mean % change from baseline | **MD-group** (n=30) Mean % change from baseline | **MDK-group** (n=38) Mean % change from baseline |
| CC ($mm^2$/kPa) | -5.9% ± 19.5 | -3.7% ± 18.6 | 2.7% ± 20.4* |
| IMT (mm) | 8.6% ± 13.5 | 4.0% ± 13.9 | 9.8% ± 9.8* |
| *significant difference with placebo after adjustment for age, weight, smoking, mean arterial pressure and heart rate (linear regression analysis Table 3a). | | | |

Table 3a

| Multivariate regression analysis of the effects of the MDK-group and the MD-group compared to placebo on the change in vessel wall characteristics after three years with the following co-variables: baseline age, weight, smoking, heart rate and mean arterial pressure. | | | |
|---|---|---|---|
| Variables | Coefficient ± SEM | P | 95% CI |
| Y= change in DC (% relative to baseline) | | | |
| X=MDK | 11.7 ± 4.9 | 0.020 | 1.9 to 21.4 |
| X=MD | 4.2 ± 5.3 | 0.430 | -6.3 to 14.8 |
| Y=change in CC (% relative to baseline) | | | |
| X=MDK | 11.1 ±4.7 | 0.019 | 1.8 to 20.3 |
| X=MD | 3.8±5.0 | 0.459 | -6.3 to 13.8 |
| Y= change in PP (% relative to baseline) | | | |
| X = MDK | -8.9 ± 4.1 | 0.034 | -17.1 to -0.70 |
| X = MD | -3.3 ± 4.5 | 0.465 | -12.1 to 5.6 |
| Y= change in IMT (% relative to baseline) | | | |
| X=MDK | 3.0 ± 3.1 | 0.345 | -3.23 to 9.15 |
| X = MD | -2.4 ± 3.3 | 0.476 | -8.9 to 4.2 |

Example 2

In this example we show that a high vitamin K diet induces disappearance of pre-existing arterial calcifications. We performed and experiment using 48 rats. Each point in the various curves shown in Figures 3-6 is the mean of six rats, which were sacrificed. The thoracic aorta was dissected, homogenized, and precipitated calcium salts were dissolved in formic acid. The total calcium concentration in the extract was assessed by atomic absorptiometry, and is expressed as microgram calcium per gram of tissue.

Figure 3 shows the data of 18 rats on a control diet (containing 5 μg/g of vitamin K). Six of them were killed at baseline, six after 6 weeks, and the last six after 12 weeks. Apart from the cellular calcium, no accretion of calcium was observed during the test period. The remaining 30 rats received a 6-week treatment with the vitamin k-antagonist warfarin in a protocol that does not influence blood clotting, but mainly affects bone and vascular vitamin K status. The protocol used involves the oral administration of a mixture of warfarin (3 mg/g of food) and vitamin K1 (1.5 mg/g of food) and is

explained extensively in: H.M.H. Spronk et al, Tissue-specific utilization of menaquinone-4 results in the prevention of arterial calcification in warfarin-treated rats, Journal of Vascular Research 2003;40:531-537. This treatment is designated as "low K". Six rats were killed after 6 weeks of treatment, and another six after 12 weeks of treatment. As can be seen from figure 4 there was a constant increase of calcium in the arterial wall, which could be identified as calcified precipitates by histochemistry, where arteries were fixed in 4% phosphate-buffered formaline and tissues were completely sectioned and every fifth section was screened for calcification using Von Kossa staining (30 minutes, 5% silver nitrate) (data not shown).

The other rats were first treated for six weeks with warfarin. One group of animals was subsequently fed the standard diet containing 5 μg vitamin K per g of food (indicated as "norm K"). Surprisingly, the calcification did not stop upon the diet change: calcification continued as in the former experiment. Apparently, the presence of calcium precipitates is a sufficient trigger to continue further calcification (see figure 5) when using a normal diet.

Finally, we used two groups of six rats who had received the first 6-week treatment with warfarin, and a high vitamin K diet in the second 6-week period. One group received a diet containing 100 μ/g of vitamin K1, the other one a diet containing 100 μg/g of vitamin K2 (menaquinone-4). Since there was no difference between the outcomes for K1 and K2, we present the means of both groups in figure 6 (designated as "high K"). The surprising conclusions were that we did not find a difference between effects of vitamin K1 and K2, whereas such differences had been found in calcification prevention. This may be related to the relatively high dose used, and different efficacies may still be found from dose-response experiments. The most surprising finding, however, was that the pre-formed calcium crystals had dissolved to a great deal, even in this relatively short period of treatment. It may be expected that after prolonged treatment all calcified precipitates will dissolve by high vitamin K intake. The dose used for dissolving calcified precipitates in the aorta was 20 times the normal dose used (compared to the recommended allowance in humans). If extrapolated to humans the effective intake of vitamin K should thus be increased from 100 μg/day to 2 mg/day. Experiments in which lower doses are used will be initiated. However, it is likely that a similar effect will be observed at lower doses, even as low as 1-2 times the normal daily intake. This turned out to be related to the relatively high dose used. The experiment was repeated at five times lower doses of K1 and MK-4. It resulted that at doses of of 20 μg/g food, K1 did not significantly decrease the rate of artery calcification, resulting in a calcium content of 2.8 μg/g of arterial tissue. Supplementing the food with similar amounts of K2 (menaquinone-4), on the other hand, resulted in tissue calcium concentrations of 0.8 μg/g arterial tissue, which was significantly below the tissue calcium content before treatment (1.7 μg/g tissue). The most surprising finding in these experiments was that the pre-formed calcium crystals had dissolved to a great deal, even in this relatively short period of treatment. It may be expected that after prolonged treatment all calcified precipitates will dissolve by high vitamin K intake. The dose used for dissolving calcified precipitates in the aorta was 4- 20 times the normal dose used (compared to the recommended allowance in humans). If extrapolated to humans the effective intake of vitamin K should thus be increased from 100 μg/day to 2 mg/day for K1 and to 400 μg/day for K2. Experiments in which still lower doses are used will be initiated. It is likely that a similar effect will be observed at lower doses of vitamin K, even as low as 1-2 times the normal daily intake. This will depend upon factors such as vitamin K absorption since synthetic supplemented vitamin K is typically absorbed better (e.g. 10-20 fold) than nutritional vitamin K (e.g. which may be bound to chloroplasts in the food matrix).

<u>Example 3</u>

In another experiment vascular calcification was induced in rats (n=30) by feeding them the previously described diet containing warfarin (3 mg/g food) and vitamin $K_1$ (1.5 mg/g food. These animals are designated as the W&K group. Control rats (n=18) received no warfarin and a normal dose of vitamin $K_1$ (5 μg/g food: ~ 10 times the minimal amount required for optimal blood coagulation). From the control group, six rats where sacrificed at start of the experiment to measure the baseline calcium content of the abdominal aorta and left carotid artery. After six weeks of treatment, 6 control rats and 6 W&K rats were sacrificed to monitor the effect of treatment. The remaining rats in the W&K group (n= 24) were subdivided into four groups of six rats for another six-week treatment. One group continued the W&K diet, whereas warfarin was discontinued in the remaining thee groups: one group received normal vitamin $K_1$ (5 μg/g food), one group received high vitamin $K_1$ (100 μg/g food: ~ 200 times the minimal required dose), and the last group received high vitamin $K_2$ (menaquinone-4, 100 μg/g food). In addition, the remaining 6 control rats continued their diet for another six weeks. The right carotid artery from all animals was dissected and used to determine arterial distensibility. Artery segments (3-4 mm) were mounted in an arteriograph (Living System Instrumentation, Burlington, USA) in which the arterial diameter could be continuously monitored. Both ends of the vessels were cannulated on 120 to 150 μm wide glass micropipettes and tied with two 17 μm thin nylon threads. Arterial segments were bathed in a 10 mL organ chamber filled with calcium-free physiological salt solution (composition in mmol/L: NaCl 144, KCl 4.7, $MgSO_4$ 1.2, $KH_2PO_4$ 1.2, HEPES 14.9, and glucose 5.5, pH 7.4) which was maintained at 37°C and gassed with 95% $O_2$ and 5% $CO_2$. After administration of 10 μmol/L Na-nitroprusside to assure maximal vasodilatation, intra-arterial pressure was gradually increased from 10 to 200 mm Hg. Arterial distensibility or the relative change in arterial lumen volume induced by a given

increase in pressure, was estimated by: $DC = \Delta A/A_{n-1} * \Delta P$ (DC = distensibility, A = area, P = pressure). Table 4 summarizes pressure-diameter relation of isolated carotid arteries during maximal vasodilatation (five groups from the 7-12 weeks experiment). Within a physiological pressure range (80 - 120 mm Hg) the arterial distensibility was significantly smaller in the W&K and normal K groups than in the control and high vitamin K groups. At 100 mm Hg distensibility averaged 0.59 ± 0.31, 0.34 ± 0.09, 0.41 ± 0.08, 0.60 ± 0.12, and 0.80 ± 0.07 mm $Hg^{-1}$ for control, W&K, normal $K_1$, high $K_1$ and high $K_2$, respectively (significance $p < 0.02$ compared to W&K). At blood pressure levels > 100 mm Hg the distensibility in the $K_2$ group was substantially and significantly ($p < 0.01$) higher than in either the high $K_1$ group or in the control animals.

Collectively these findings indicate that the W&K treatment increased the arterial stiffness and that this was reversed only by the high vitamin K intake and not by the normal vitamin K diet, and that $K_2$ had a more pronounced effect than $K_1$. We measured the arterial distensibility as a clinical parameter of vascular elasticity. The experiment shown in Table 4 demonstrates that warfarin induced stiffening of the arterial vessel wall. This is consistent with work from Essahili *et al* (Essalihi R, Dao HH, Yamaguchi N, Moreau P. A new model of isolated systolic hypertension induced by chronic warfarin and vitamin K1 treatment. Am J Hypertens. 2003;16:103-10) who showed that warfarin treatment resulted in increases of aortic pulse pressure, pulse pressure, and systolic blood pressure. In our model, normal vitamin $K_1$ in the diet was not capable of affecting vascular elasticity. Surprisingly, however, during the high vitamin K diet (both $K_1$ and $K_2$) the vascular properties that were lost by warfarin-induced calcification were restored.

Table 4

| Blood pressure | Control | K&W | Normal $K_1$ | High $K_1$ | High $K_2$ |
|---|---|---|---|---|---|
| Mm Hg | n = 6 | n = 6 | n = 6 | n = 6 | n = 6 |
| | Mean ± SD | Mean ± SD | Mean ± SD | Mean ± SD | Mean ± SD |
| 80 | 0.52 ± 0.03 | 0.47 ± 0.11 | 0.42 ± 0.13 | 0.54 ± 0.17 | 0.47 ± 0.24 |
| 85 | 0.65 ± 0.07 | 0.51 ± 0.14 | 0.41 ± 0.15 | 0.45 ± 0.17 | 0.77 ± 0.09 |
| 90 | 0.40 ± 0.04 | 0.49 ± 0.14 | 0.52 ± 0.21 | 0.74 ± 0.18 | 0.70 ± 0.19 |
| 95 | 0.56 ± 0.14 | 0.49 ± 0.24 | 0.45 ± 0.18 | 0.45 ± 0.04 | 0.61 ± 0.17 |
| 100 | 0.59 ± 0.31 | 0.34 ± 0.09 | 0.41 ± 0.08 | 0.60 ± 0.12 | 0.80 ± 0.07 |
| 105 | 0.53 ± 0.28 | 0.33 ± 0.25 | 0.38 ± 0.17 | 0.54 ± 0.12 | 0.57 ± 0.04 |
| 110 | 0.56 ± 0.12 | 0.26 ± 0.17 | 0.31 ± 0.25 | 0.41 ± 0.19 | 0.83 ± 0.10 |
| 115 | 0.37 ± 0.03 | 0.17 ± 0.15 | 0.23 ± 0.14 | 0.33 ± 0.17 | 0.56 ± 0.12 |
| 120 | 0.28 ± 0.03 | 0.11 ± 0.09 | 0.15 ± 0.12 | 0.22 ± 0.16 | 0.48 ± 0.08 |

Table 4: Effects of vitamin K-status on the mechanical properties of isolated rat carotid arteries. The pressure-diameter relationship was monitored for animals at the 12-week time point, receiving the control diet (n = 6), those of the W&K diet (n = 6), on normal vitamin K (n= 6) and animals on either high vitamin $K_1$ or $K_2$ (n = 6 per group). The arterial diameter is expressed as a function of increasing pressure. Data are presented as mean ± SD.

The animal model we used mimics arterial media sclerosis (also known as Mönckeberg's sclerosis). Media sclerosis is particularly common in diabetes mellitus, end-stage renal disease, and aging. Notably patients with chronic kidney disease (CKD) are at high risk of cardiovascular disease. These patients often receive a high calcium diet (to complex phosphate), vitamin D, and warfarin (to prevent thrombotic events). Recently it was demonstrated, however, that each of these treatments is associated with an increased risk for arterial calcification. Given the fact that arterial calcifications are predictive of cardiovascular events, regression of arterial calcification by vitamin K-intake may help reduce the risk for death in patients with CKD and coronary artery disease. Obviously such treatment is only possible in patients not receiving oral anticoagulant treatment.

## Claims

1. Use of vitamin K for the preparation of a pharmaceutical or nutritional formulation for reversing calcification of a blood vessel in a mammal suffering from calcification of a blood vessel, when administered to the mammal an effective amount of said vitamin K to reverse calcification of the blood vessel.

2. Use according to claim 1, wherein reversing calcification of a blood vessel includes the removal of pre-existing

calcium deposits in and/or on the blood vessel.

3. Use according to claim 1, wherein calcification of a blood vessel is associated with a disease selected from the group consisting of arteriosclerosis, including Mönckeberg's sclerosis, osteoarthritis, inflammation-induced calcification, including Bechterev's disease, tumor-induced calcification, skin calcification including pseudo-xanthoma elasticum (PXE), and calcifylaxis in end-stage renal disease.

4. Use according to claim 1, wherein the vitamin K is contained in a food or beverage product or a dietary supplement.

5. Use according to claim 1, wherein the formulation further comprises vitamin D.

6. Use according to claim 1, wherein the calcification is of the tunica media of the aorta or common carotid artery.

7. Use according to claim 1, wherein the vitamin K comprises vitamin $K_1$ (phylloquinone), vitamin $K_2$ (menaquinone) or a combination thereof.

8. Use according to claim 1, wherein the daily dosage of vitamin K is 50 $\mu$g - 2000 $\mu$g.

9. Use according to claim 5, wherein the vitamin D is vitamin $D_3$ (cholecalciferol).

10. Use according to claim 1, wherein the mammal is a human.

**Patentansprüche**

1. Verwendung von Vitamin K für die Herstellung einer pharmazeutischen oder Ernährungs-Formulierung zum Umkehren der Calcifizierung eines Blutgefäßes in einem Säuger, der an einer Calcifizierung eines Blutgefäßes leidet, wenn die Formulierung an den Säuger in einer wirksamen Menge des Vitamins K zum Umkehren der Calcifizierung des Blutgefäßes verabreicht wird.

2. Verwendung nach Anspruch 1, worin ein Umkehren der Calcifizierung eines Blutgefäßes das Entfernen vorher existierender Calcium-Ablagerungen in und/oder an dem Blutgefäß einschließt.

3. Verwendung nach Anspruch 1, worin eine Calcifizierung eines Blutgefäßes assoziiert ist mit einer Krankheit, die gewählt ist aus der Gruppe, die besteht aus Arteriosklerose, einschließlich Mönckeberg'scher Sklerose, Osteoarthritis, Entzündungs-induzierter Calcifizierung, einschließlich Bechterev'scher Krankheit, Tumor-induzierter Calcifizierung, Haut-Calcifizierung einschließlich Pseudoxanthoma Elasticum (PXE) und Calciphylaxie bei Nierenkrankheiten im End-Stadium.

4. Verwendung nach Anspruch 1, worin das Vitamin K in einem Lebensmittel- oder Getränkeprodukt oder in einem Nahrungsergänzungsmittel enthalten ist.

5. Verwendung nach Anspruch 1, worin die Formulierung weiter Vitamin D umfasst.

6. Verwendung nach Anspruch 1, worin die Calcifizierung eine Calcifizierung der Tunica media der Aorta oder der Arteria carotis communis ist.

7. Verwendung nach Anspruch 1, worin das Vitamin K Vitamin $K_1$ (Phyllochinon), Vitamin $K_2$ (Menachinon) oder eine Kombination daraus umfasst.

8. Verwendung nach Anspruch 1, worin die tägliche Dosierung von Vitamin K 50 $\mu$g - 2.000 $\mu$g beträgt.

9. Verwendung nach Anspruch 5, worin das Vitamin D Vitamin $D_3$ (Cholecalciferol) ist.

10. Verwendung nach Anspruch 1, worin der Säuger ein Mensch ist.

## Revendications

1. Utilisation de la vitamine K pour la préparation d'une formulation pharmaceutique ou nutritionnelle pour inverser la calcification d'un vaisseau sanguin chez un mammifère soufrant de calcification d'un vaisseau sanguin, lors d'une administration chez le mammifère d'une quantité efficace de vitamine K pour inverser la calcification du vaisseau sanguin.

2. Utilisation selon la revendication 1, dans laquelle le fait d'inverser la calcification d'un vaisseau sanguin comprend le retrait des dépôts préexistants de calcium dans et/ou sur le vaisseau sanguin.

3. Utilisation selon la revendication 1, dans laquelle la calcification d'un vaisseau sanguin est associée à une maladie inclue dans le groupe comprenant l'artériosclérose, comprenant la sclérose de Mönckeberg, l'ostéoartrite, la calcification induite par inflammation, comprenant la maladie de Bechterev, la calcification induite par tumeur, la calcification de la peau comprenant le pseudoxanthome élastique (PXE), et la calciphylaxie en phase terminale de la maladie rénale.

4. Utilisation selon la revendication 1, dans laquelle la vitamine K est contenue dans un produit alimentaire ou une boisson ou un supplément diététique.

5. Utilisation selon la revendication 1, dans laquelle la formulation comprend en outre la vitamine D.

6. Utilisation selon la revendication 1, dans laquelle la calcification est une calcification de la media de l'aorte ou de l'artère carotide primitive.

7. Utilisation selon la revendication 1, dans laquelle la vitamine K comprend de la vitamine $K_1$ (phylloquinone), de la vitamine $K_2$ (ménaquinone) ou une combinaison de ces deux éléments.

8. Utilisation selon la revendication 1, dans laquelle la dose quotidienne de vitamine K est de 50 $\mu$g - 2000 $\mu$g.

9. Utilisation selon la revendication 5, dans laquelle la vitamine D est de la vitamine $D_3$ (cholécalciférol).

10. Utilisation selon la revendication 1, dans laquelle le mammifère est un humain.

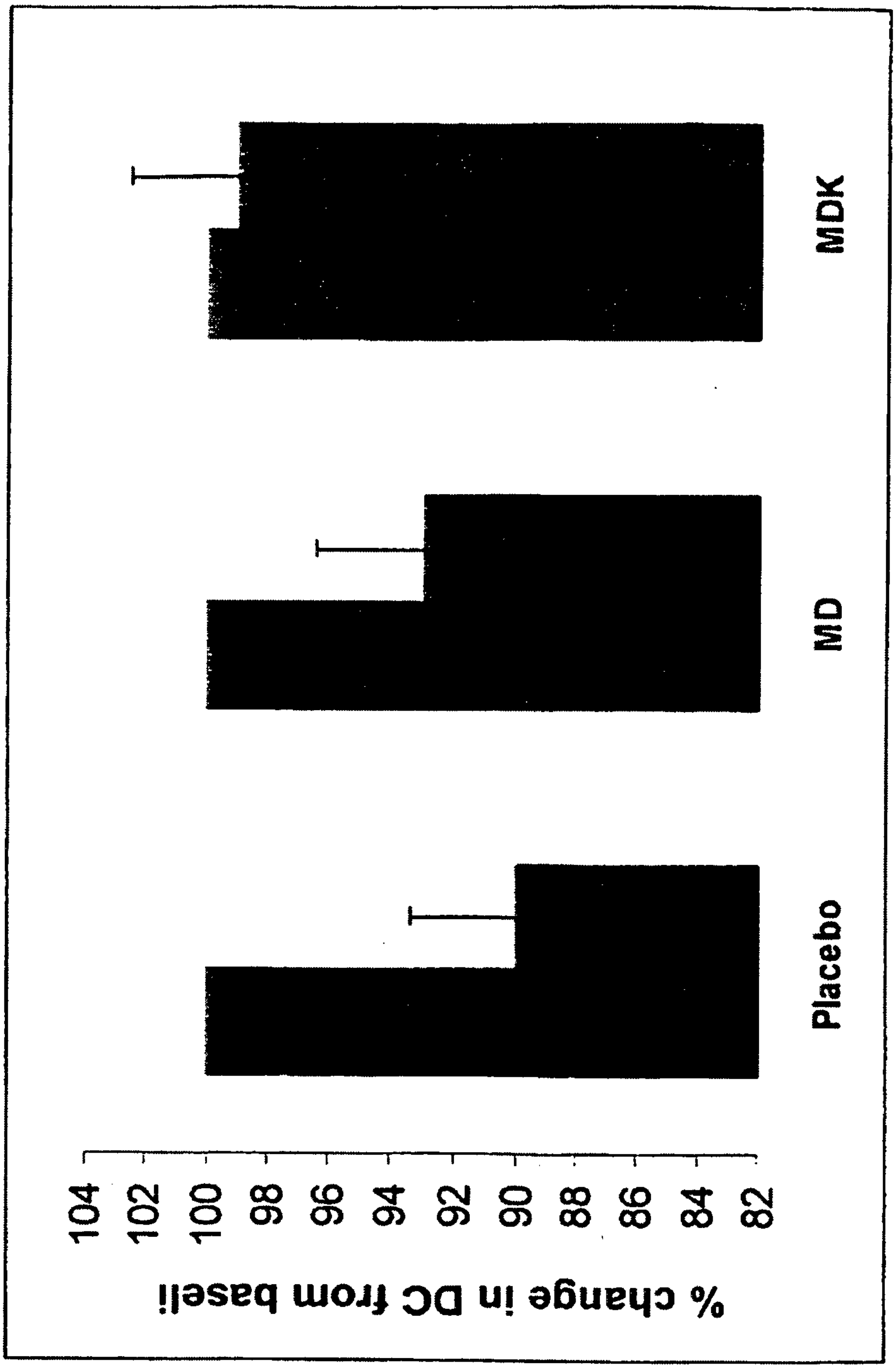
% change in DC from baseli
104
102
100
98
96
94
92
90
88
86
84
82
Placebo
MD
MDK

FIG. 1

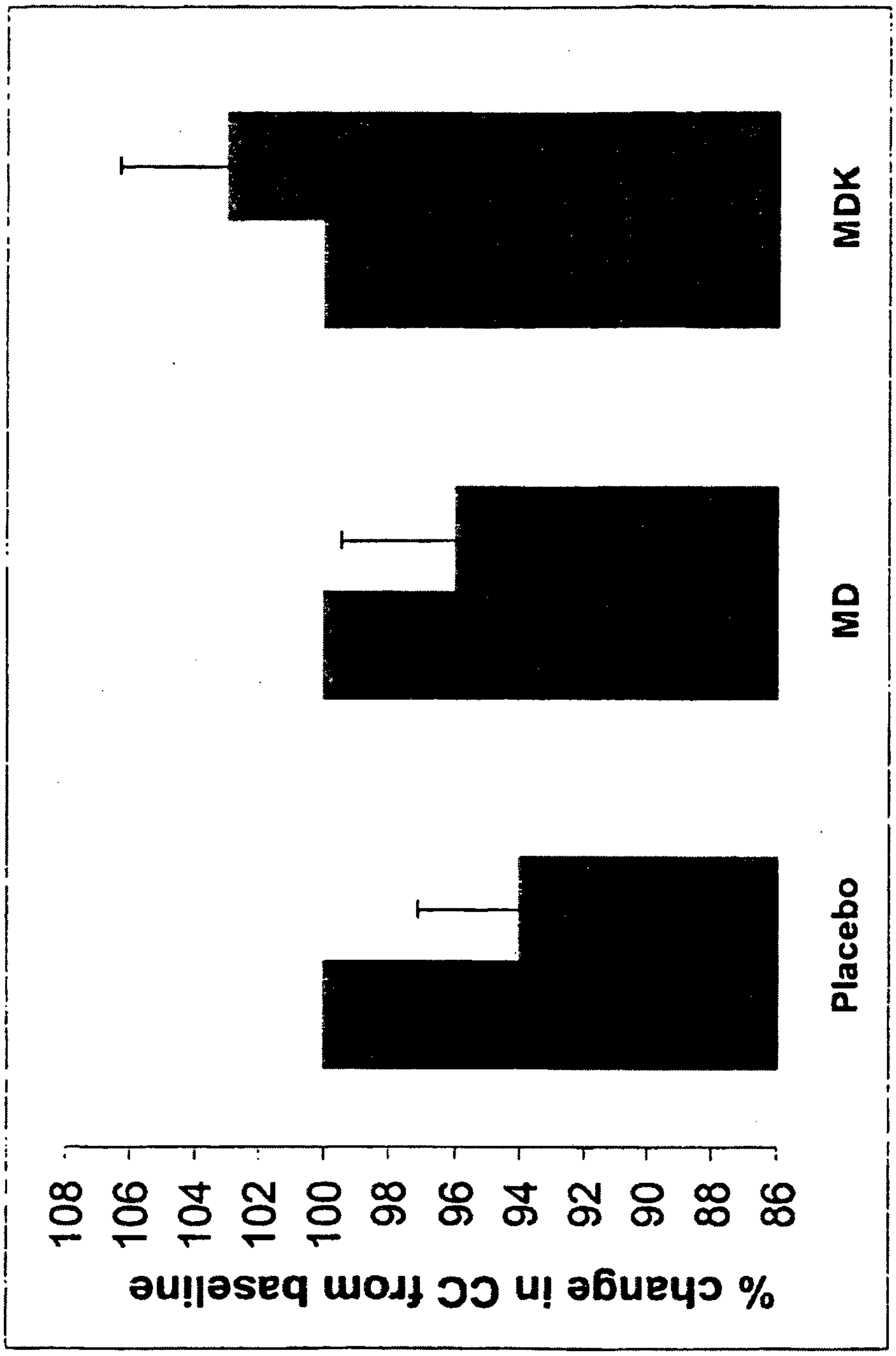
% change in CC from baseline
108
106
104
102
100
98
96
94
92
90
88
86
Placebo
MD
MDK

FIG. 2

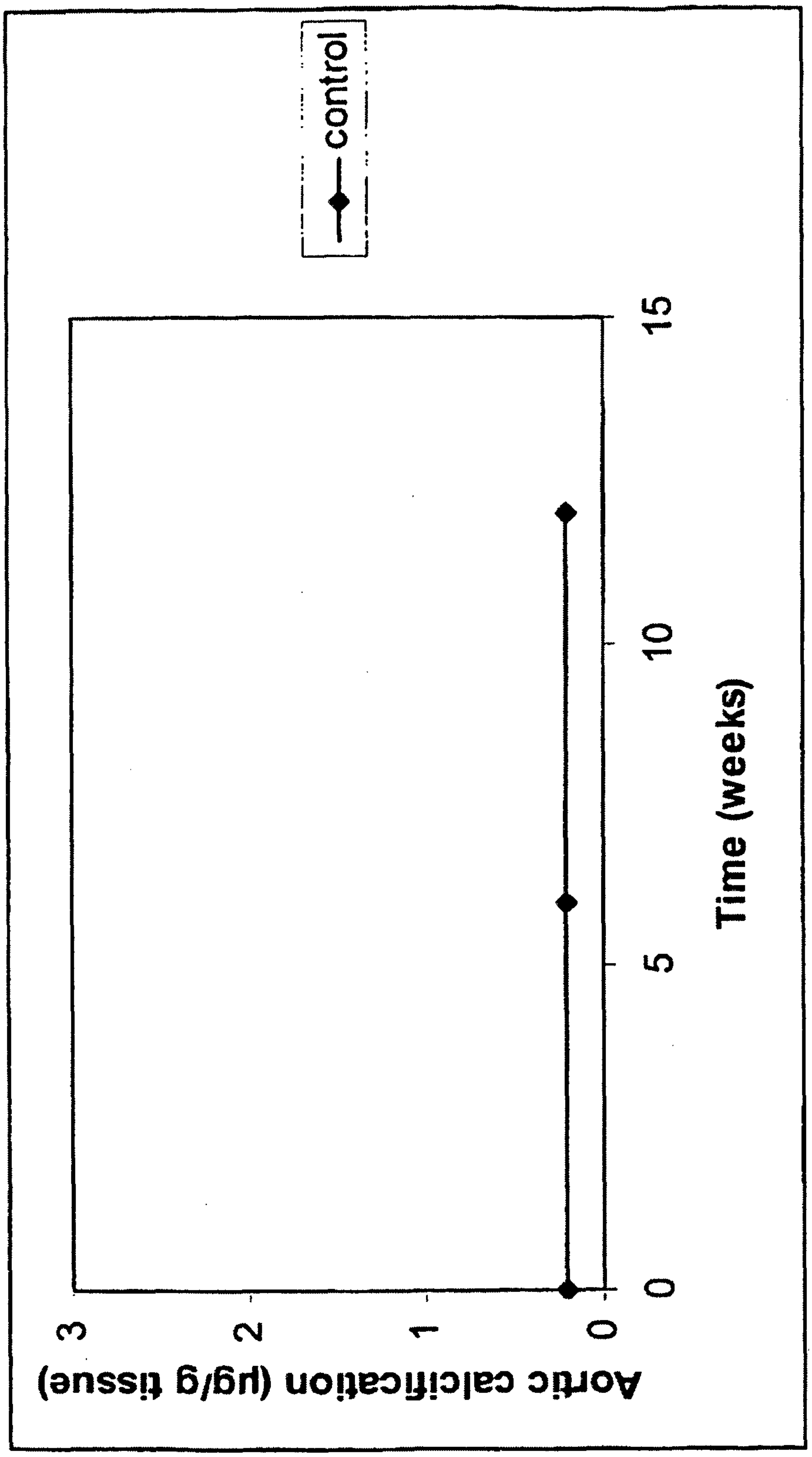
control
Aortic calcification (µg/g tissue)
0
1
2
3
0
5
10
15
Time (weeks)

FIG. 3

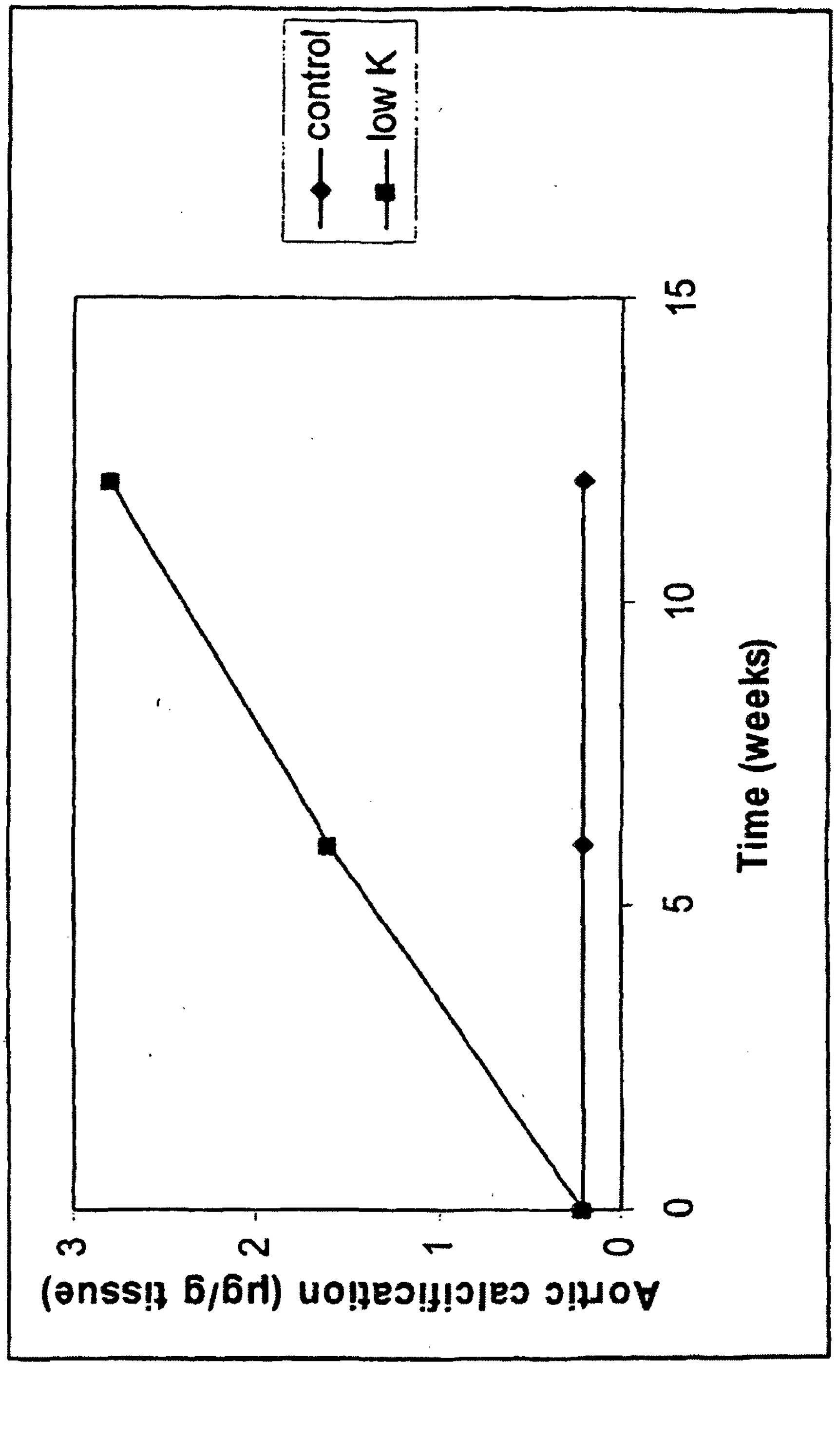
Aortic calcification (µg/g tissue)
0
1
2
3
0
5
10
15
Time (weeks)
control
low K

FIG. 4

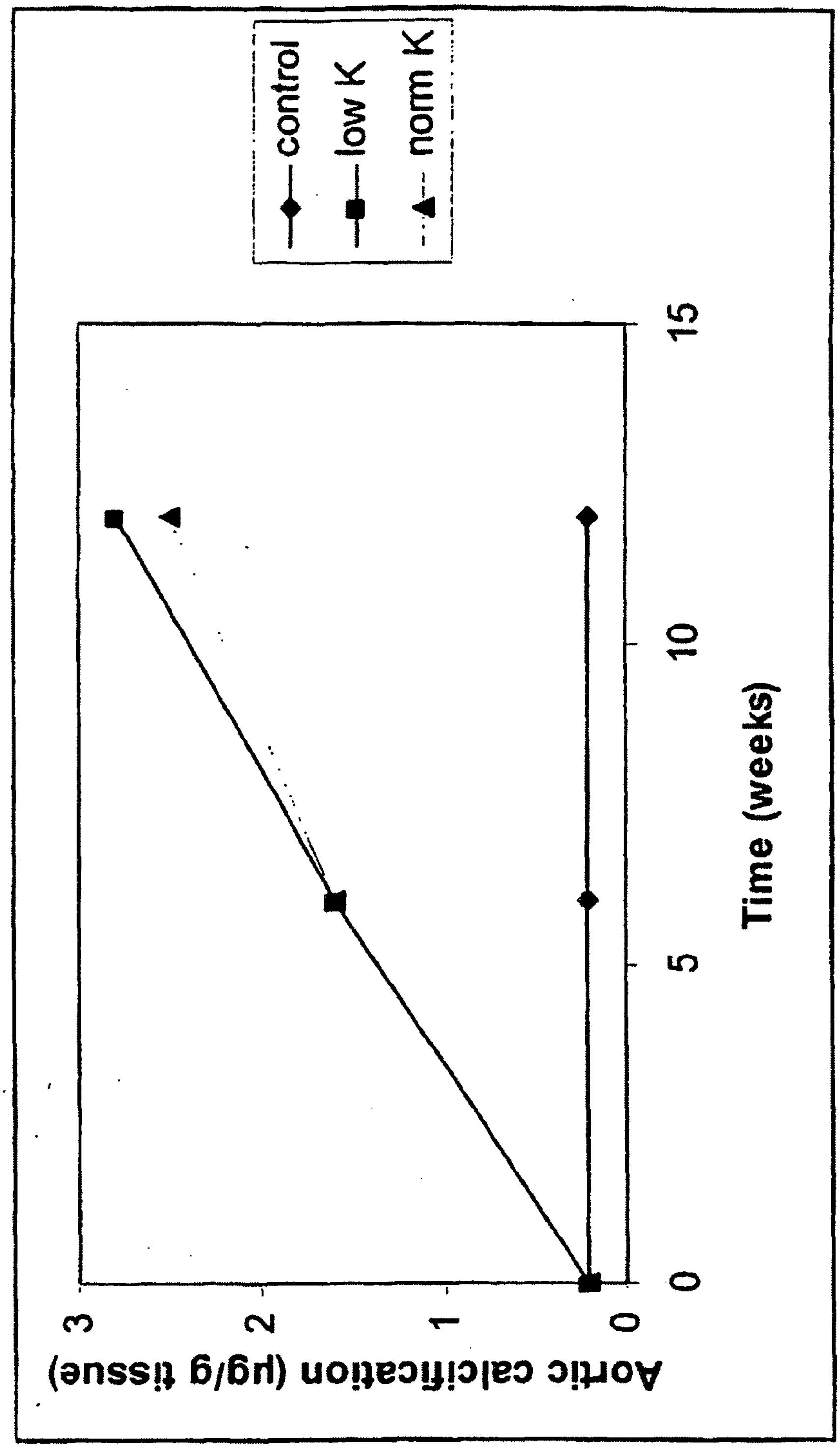

Aortic calcification (µg/g tissue)
0
1
2
3
0
5
10
15
Time (weeks)
control
low K
norm K


FIG. 5

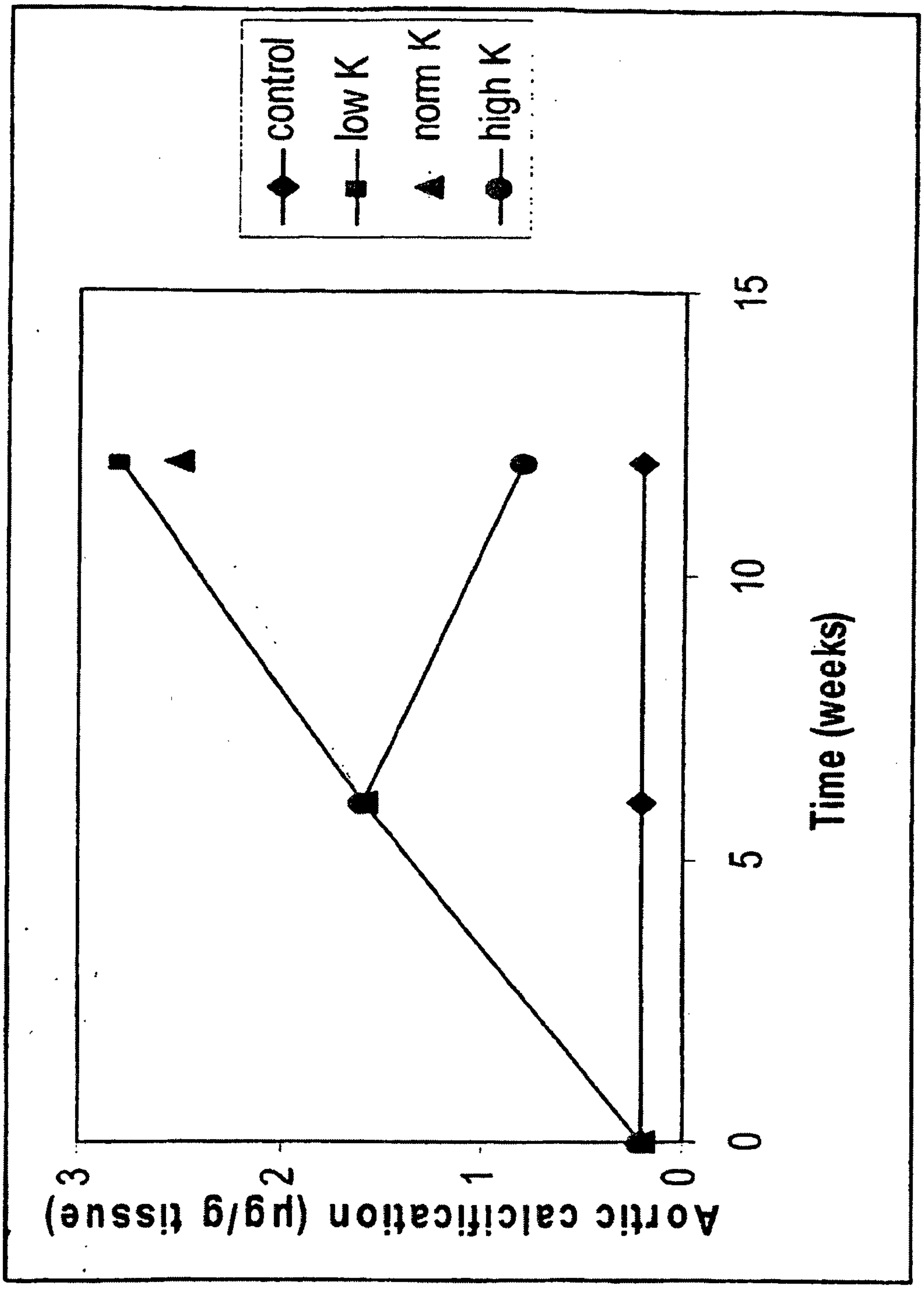
Aortic calcification (µg/g tissue)
0
1
2
3
0
5
10
15
Time (weeks)
control
low K
norm K
high K

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0679394 A
- WO 2004019923 A
- EP 1153548 A


### Non-patent literature cited in the description


- *Jpn. J. Pharmacol.,* 1997, vol. 75, 135-143
- **HOEKS AP et al.** *Ultrasound Med Biol,* 1990, vol. 16, 121-8
- **KOOL MJF et al.** *Cardiovascular Research,* 1994, vol. 28, 610-614
- **H.M.H. SPRONK et al.** Tissue-specific utilization of menaquinone-4 results in the prevention of arterial calcification in warfarin-treated rats. *Journal of Vascular Research,* 2003, vol. 40, 531-537
- **ESSALIHI R ; DAO HH ; YAMAGUCHI N ; MOREAU P.** A new model of isolated systolic hypertension induced by chronic warfarin and vitamin K1 treatment. *Am J Hypertens.,* 2003, vol. 16, 103-10